# EUROPEAN PATENT APPLICATION

(11) **EP 4 737 486 A1**
(43) Date of publication of application: **06.05.2026**
(21) Application number: 24830112.9
(22) Date of filing: 23.04.2024
(51) Int. Cl.: C08B 37/08, A61K 31/728, A61P 31/14

(54) **HYALURONIC ACID DERIVATIVE, AND PREPARATION METHOD THEREFOR AND USE THEREOF**

(30) Priority: 29.06.2023 CN 202310778756
(71) Applicant: Five Hertz Innovation (Xiamen) Medical Technology Co., Ltd., Xiamen, Fujian 361000 (CN)
(72) Inventor: ASARI, Akira, Xiamen, Fujian 361000 (CN); ZHANG, Libo, Xiamen, Fujian 361000 (CN)
(74) Representative: Gille Hrabal Partnerschaftsgesellschaft mbB Patentanwälte
(86) International application number: PCT/CN2024/089382
(87) International publication number: WO 2025/001463

(57) **Abstract**

The present invention relates to a hyaluronic acid derivative as shown in formula I, and a preparation method thereof and the use thereof in the preparation of an anti-infection drug. The structure of the compound or the pharmaceutically acceptable salt thereof of the present invention is significantly different from that of commercially available hyaluronic acid substances such as TCIsHA and R&D HA, and the compound or the pharmaceutically acceptable salt thereof can form a molecular structure recognized by HepSS-1. The molecule with the new structure can achieve an anti-infection effect by means of inhibiting the binding of SARS-Cov-2 spike protein to ACE2 of a host cell, and is less likely to cause serious adverse reactions clinically, such as bleeding and thrombocytopenia, as compared with heparin.

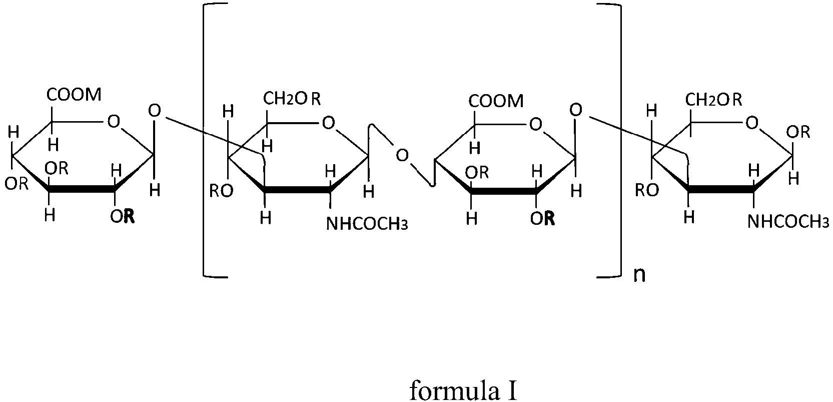

## Description

The present application claims priority to the prior application with the patent application No. 202310778756.8 entitled "HYALURONIC ACID DERIVATIVE, AND PREPARATION METHOD THEREFOR AND USE THEREOF" and filed to the China National Intellectual Property Administration on June 29, 2023, which is incorporated herein by reference in its entirety.

### TECHNICAL FIELD

The present disclosure relates to the field of pharmaceutical chemistry, and particularly, to a hyaluronic acid derivative, a preparation method therefor, and use thereof.

### BACKGROUND

Hyaluronic acid (HA) is a glycosaminoglycan composed of D-glucuronic acid and N-acetylglucosamine linked alternately by β-1,4-glycosidic bonds and β-1,3-glycosidic bonds, and is an important component in the natural extracellular matrix. Hyaluronic acid substances may have different pharmacological activity depending on molecular weight and modified groups, such that they can be widely applied to the fields of cosmetics, food, and pharmaceuticals.

The high water absorption capacity and high viscoelasticity of hyaluronic acid confer unique properties in biomaterials, making it suitable for various medical and pharmaceutical applications. Various hyaluronic acid products can be found in daily life. For example, since hyaluronic acid can retain water, it is used in some cosmetics to maintain youthful and fresh skin. Although hyaluronic acid is abundant in the skin, the water-retaining capacity of hyaluronic acid in the skin decreases with age due to depolymerization. Therefore, retaining abundant hyaluronic acid in the skin can help maintain a youthful appearance.

In fields other than cosmetics, researchers have proposed many concepts for applying hyaluronic acid. However, there are few practical applications of hyaluronic acid in medicine, mainly in the treatment of osteoarthritis and in ophthalmic medical devices. Heparin is also a glycosaminoglycan composed of hexuronic acid and N-acetylglucosamine linked alternately by 1,4-glycosidic bonds, and has been widely used as an anticoagulant drug in clinical practice. Some modified hyaluronic acids exhibit an anticoagulant effect similar to that of heparin. However, due to the anticoagulant activity of heparin and analogs thereof, serious adverse reactions such as bleeding and thrombocytopenia are easily caused.

### SUMMARY

To ameliorate the technical problems existing in the prior art, in a first aspect, the present disclosure provides a compound of formula I or a pharmaceutically acceptable salt thereof, wherein the structure of formula I is shown as follows: 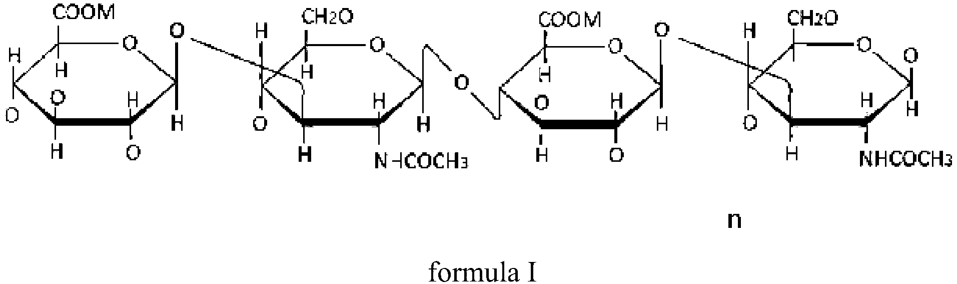
wherein in formula I, R is identical or different and is each independently selected from H and SO₃H;
M is selected from a proton and a monovalent cation;
n is selected from 3-9.

According to an embodiment of the present disclosure, n is selected from 3, 4, 5, 6, 7, 8, and 9.

According to an embodiment of the present disclosure, the monovalent cation is selected from Na⁺ and K⁺.

According to an embodiment of the present disclosure, the weight-average molecular weight of formula I is less than 10000. In some embodiments, the weight-average molecular weight of formula I is 2480-6590.

According to an embodiment of the present disclosure, in formula I, at least part of R is selected from SO₃H (that is, R is not all H). In some embodiments, in formula I, the degree of sulfation is less than or equal to 4.2; in some embodiments, in formula I, the degree of sulfation is greater than 3.65, and preferably, the degree of sulfation is greater than or equal to 3.75; in some embodiments, in formula I, the degree of sulfation is less than or equal to 4.2 and greater than 3.65; in some embodiments, in formula I, the degree of sulfation is less than or equal to 4.2 and greater than or equal to 3.75; in some embodiments, in formula I, the degree of sulfation is less than or equal to 4.0 and greater than or equal to 3.75; for example, the degree of sulfation is selected from 3.70, 3.75, 3.80, 3.85, 3.90, 3.95, 4.00, 4.05, 4.10, 4.05, 4.10, 4.15, and 4.20.

According to an embodiment of the present disclosure, in formula I, NHCOCH₃ is not sulfated.

According to an embodiment of the present disclosure, the structure of formula I can be recognized by HepSS-1.

In a second aspect, the present disclosure provides a preparation method for the compound of formula I or the pharmaceutically acceptable salt thereof, comprising the following steps:
(1) mixing water with hyaluronic acid or a water-soluble salt thereof;
(2) adding a sulfating agent and reacting;
(3) adding ethanol, mixing, and centrifuging;
(4) removing supernatant and adding water, adding ethanol, mixing, and centrifuging;
(5) removing supernatant and allowing to stand for 4-24 hours; and
(6) adding water, filtering, and lyophilizing to obtain a product.

According to an embodiment of the present disclosure, the sulfating agent is selected from coordination complexes of sulfur trioxide, preferably pyridine coordination complexes of sulfur trioxide.

According to an embodiment of the present disclosure, the hyaluronic acid or the water-soluble salt thereof is selected from Hyalonano, and preferably, the hyaluronic acid or the water-soluble salt thereof is purchased from Kewpie.

According to an embodiment of the present disclosure, in step (1), 0.5-3 g of hyaluronic acid or a water-soluble salt thereof is mixed with each 1 mL of water; preferably, 0.8-1.2 g of hyaluronic acid or a water-soluble salt thereof is mixed with each 1 mL of water; and for example, 1 g of hyaluronic acid or a water-soluble salt thereof is mixed with each 1 mL of water.

According to an embodiment of the present disclosure, the water-soluble salt is selected from sodium hyaluronate.

According to an embodiment of the present disclosure, in step (2), the reaction is performed at a temperature of 0-20 °C, e.g., 0 °C, 1 °C, 2 °C, 3 °C, 4 °C, 5 °C, 6 °C, 7 °C, 8 °C, 9 °C, 10 °C, 11 °C, 12 °C, 13 °C, 14 °C, 15 °C, 16 °C, 17 °C, 18 °C, 19 °C, or 20 °C, preferably, 3-10 °C; and the reaction is performed for 24-100 hours, preferably 36-90 hours, e.g., 36 hours, 48 hours, 60 hours, 72 hours, or 84 hours.

According to an embodiment of the present disclosure, the sulfating agent and the hyaluronic acid or the water-soluble salt thereof are in a mass ratio of 0.5-6:100, preferably 1-3:100, e.g., 1.5:100, 2.0:100, 2.5:100, 3.0:100, 3.5:100, or 4.0:100.

According to an embodiment of the present disclosure, in step (3), the addition amount of ethanol is: 10-50 mL of ethanol per 1 g of hyaluronic acid or the water-soluble salt thereof, and preferably 20-30 mL of ethanol, e.g., 25 mL of ethanol, is added to each 1 g of hyaluronic acid or the water-soluble salt thereof.

According to an embodiment of the present disclosure, in step (4), the addition amount of water is: 0.5-2 mL of water, preferably 1 mL of water, per 1 g of hyaluronic acid or the water-soluble salt thereof; the addition amount of ethanol is: 20-30 mL of ethanol, preferably 25 mL of ethanol, per 1 g of hyaluronic acid or the water-soluble salt thereof. According to an embodiment of the present disclosure, in step (3) and step (4), the centrifugation is performed at 0-10 °C at 2000-4000 rpm for 5-30 minutes; preferably, the centrifugation is performed at a temperature of 3-6 °C, e.g., 4 °C; preferably, the centrifugation is performed at a rotation speed of 3000 rpm; preferably, the centrifugation is performed for 10-20 minutes, e.g., 15 min.

According to an embodiment of the present disclosure, in step (5), the standing is performed at a temperature of -20-5 °C, preferably -10-4 °C; the standing is performed for 6-12 hours preferably.

According to an embodiment of the present disclosure, in step (6), the addition amount of water is: 0.5-2 mL of water, preferably 1 mL of water, per 1 g of hyaluronic acid or the water-soluble salt thereof.

According to an embodiment of the present disclosure, in step (6), a disc filter is used, and preferably, a 0.45 µm disc filter is used.

In a third aspect, the present disclosure further provides a composition comprising the compound of formula I or the pharmaceutically acceptable salt thereof.

According to an embodiment of the present disclosure, the composition further comprises a pharmaceutically acceptable carrier.

In a fourth aspect, the present disclosure provides use of the compound of formula I or the pharmaceutically acceptable salt thereof or the composition for the manufacturing of a medicament for treating an infection.

According to an embodiment of the present disclosure, the infection is selected from an infection caused by coronavirus (COVID-19); the coronavirus is selected from SARS-CoV-2.

According to an embodiment of the present disclosure, the formulation of the medicament may be administered topically via the skin, for example, the formulation being selected from a cream, a patch, an ointment, a cream preparation, a gel, a spray, and the like, and may also be administered orally (i.e., an oral formulation), for example, in a solid form, the formulation being selected from a tablet, a granule, a capsule, a pill, a dripping pill, and the like. In some embodiments, the formulation of the medicament may further be selected from a liquid or semi-solid dosage form (a liquid, a suspension, a solution, a dispersion, an ointment, a cream, an emulsion, a microemulsion, a spray, a patch, and a drop). Injectable formulations, parenteral formulations, inhalation formulations, sublingual formulations, nasal formulations, and the like are also within the scope of the present disclosure.

The formulation further comprises a physiologically acceptable carrier (e.g., a biocompatible material), for example, optionally comprising a surfactant, an excipient, a humectant, an emulsification accelerant, a suspending agent, a salt or buffering agent for adjusting osmotic pressure, a colorant, an essence, a stabilizer, a bactericide, a preservative, or other conventional supplements.

In a fifth aspect, the present disclosure provides a method for treating an infection, comprising administering to a patient a therapeutically effective amount of the compound of formula I or the pharmaceutically acceptable salt thereof or the composition described herein.

According to an embodiment of the present disclosure, the infection is selected from an infection caused by coronavirus (COVID-19).

### Beneficial Effects of Present Disclosure

It is found in the present disclosure that the compound of formula I or the pharmaceutically acceptable salt thereof has a structure that is significantly different from commercially available hyaluronic acid substances such as TCIs HA and R&D HA, and can form a molecular structure recognizable by HepSS-1. Such a structurally novel molecule can provide anti-infective effects by inhibiting the binding of the SARS-CoV-2 spike protein to ACE2 on host cells, and, compared with heparin, is less likely to cause serious adverse clinical reactions such as bleeding and thrombocytopenia.

### BRIEF DESCRIPTION OF THE DRAWINGS

FIG. 1 schematically illustrates the ¹H NMR nuclear magnetic resonance spectrum of sHA;
FIG. 2 schematically illustrates the results of the HepSS-1 recognition assay;
FIG. 3 schematically illustrates the HPLC profile of sHA synthesized according to the present disclosure, wherein the numbers represent the number of saccharide residues and the arrows represent the same number of disaccharide units (wherein the numbers such as 2, 4, 6, and the like represent the number of saccharide residues; the solid arrows indicate sulfation screening to higher MV; the dashed arrow indicates that the maximum MW limit of sHA is almost the same as that of Fragmin);
FIG. 4 schematically illustrates the binding assay results of the compound of the present disclosure to the SARS-CoV-2 spike protein; and
FIG. 5 schematically illustrates the prothrombin time for sHA and control substances.

### DETAILED DESCRIPTION

The technical solutions of the present disclosure are further described in detail below with reference to specific examples. It will be appreciated that the following examples are merely exemplary illustrations and explanations of the present disclosure and should not be construed as limiting the claimed scope of the present disclosure. All techniques implemented on the basis of the content described above of the present disclosure fall within the claimed scope of the present disclosure.

Unless otherwise stated, the starting materials and reagents used in the following examples are all commercially available products or can be prepared by using known methods.

### Example 1

### Preparation of Compound of the Present Disclosure

### 1.1. Preparation method for compound of formula I sHA

### 1.11. Preparation of compound of formula I sHA-1

To 20 mL of deionized water was added 20 g of hyaluronic acid (trade name: Hyalonano, Kewpie 38399), and the mixture was mixed. Subsequently, 0.3 g of pyridine-sulfur trioxide coordination complex (purchased from FUJIFILM Wako Pure Chemical Corporation) was added, and the mixture was mixed at 4 °C for 72 h. 500 mL of ethanol was added, and the mixture was mixed and centrifuged (at 4 °C and 3000 rpm for 15 min). The supernatant was removed, 20 mL of deionized water was added, and 500 mL of ethanol was added. The mixture was mixed and centrifuged (at 4 °C and 3000 rpm for 15 min). The supernatant was removed, 20 mL of deionized water was added, and 500 mL of ethanol was added. The mixture was mixed and centrifuged (at 4 °C and 3000 rpm for 15 min). The supernatant was removed and stored in a refrigerator overnight. 20 mL of deionized water was added, and the solution was applied to a disc filter (0.45 µm: ADVANTEC) and was then lyophilized to obtain 1.73 g of product (i.e., sHA-1).

1.12. Referring to the process in 1.11, compounds of formula I sHA-2 to sHA-7 were prepared by selecting different mass ratios of the pyridine-sulfur trioxide coordination complex to hyaluronic acid.

### 1.2. Structural identification of compound of formula I sHA

### 1.2.1. Detection method

(1) ¹H NMR
(2) HPLC
(3) HepSS-1 recognition (based on ELISA)

The samples used are shown in the following table:

| | |
|---|---|
| TCIs HA | Highly sulfated hyaluronic acid (manufactured by Tokyo Chemical Industry Co., Ltd. (TCI), CAS RN: 95507-66-7, product model: H1739), MW: 30000-70000 |
| sHA-1 to sHA-7 | Preparation process according to section 1.1 of Example 1 |
| R&D HA | Hyaluronic acid: 75-350 kDa, GLR004 |

The highly sulfated hyaluronic acid TCIs HA may also be prepared with reference to the literature [Highly sulfated hyaluronic acid maintains human induced pluripotent stem cells under feeder-free and bFGF-free conditions. Taichi Miuraa, Noriyuki Yuasac, Hayato Otab, Masato Habuc, Mitsuko Kawanoa, Fumiaki Nakayamaa, Shoko Nishihara. August 2019, Biochemical and Biophysical Research Communications518(3)].

The steps of the method are as follows:
each sample (20 µg/100 µL) was immobilized on a 96-well plate;
the sample was incubated with HepSS-1;
the sample was incubated with HRP-anti-mouse IgM;
the sample was incubated with 3,3'-diaminobenzidine (DAB) and 30% hydrogen peroxide; and
detection was performed using an iMark plate Reader at a wavelength of 405 nm.

### 1.2.2. Analysis of identification results

Upon identification, the structures of compounds sHA-1 to sHA-7 are shown as follows:

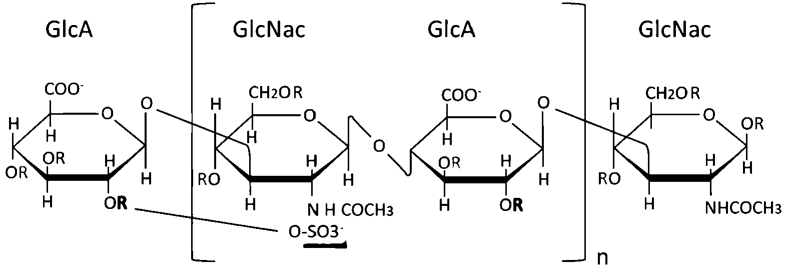

In the structures described above, R is H or SO₃H; GlcA represents glucuronic acid; GlcNac represents N-acetylglucosamine; and n = 3.

| Compound | Mass ratio of pyridine-sulfur trioxide coordination complex to hyaluronic acid | Degree of sulfation |
|---|---|---|
| sHA-1 | 1.5:100 | 3.7 |
| sHA-2 | 2.0:100 | 3.85 |
| sHA-3 | 2.5:100 | 3.9 |
| sHA-4 | 3.0:100 | 4.05 |
| sHA-5 | 3.5:100 | 4.1 |
| sHA-6 | 4.0:100 | 4.15 |
| sHA-7 | 4.5:100 | 4.2 |

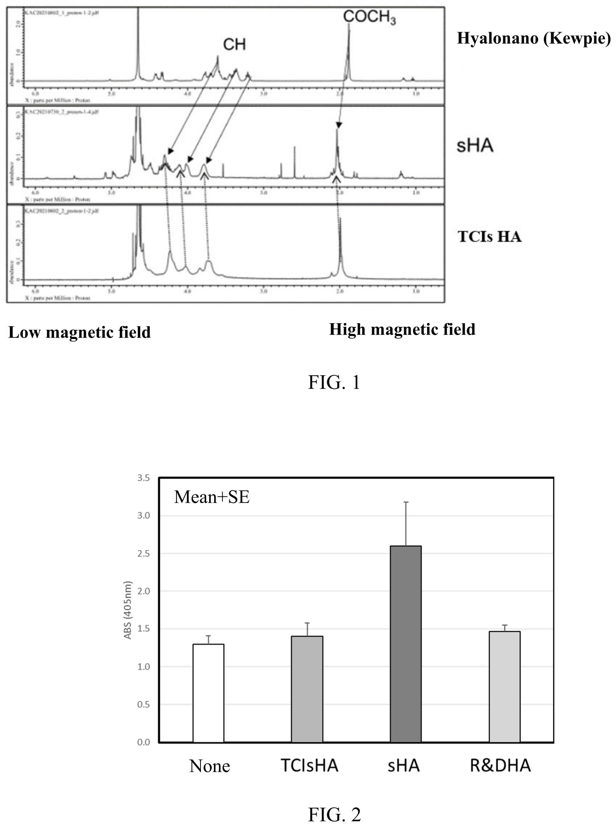

(1) As identified by ¹H NMR and HPLC, in the structures of sHA-1 to sHA-7 described above, n was 3 (8 mers), the weight-average molecular weight (MW) was 2480, and the degree of sulfation (i.e., the number of [SO₃⁻] groups per disaccharide) was less than or equal to 4.2 and greater than 3.65 (wherein the degree of sulfation of completely sulfated disaccharides at both ends was counted as 5). In TCIs HA, the degree of sulfation was 3.65.
(2) ¹H NMR showed that in the structures of sHA-1 to sHA-7 described above, the NHCOCH₃ of GlcNac was not substituted by SO₃-, and however, the sHA described above could be recognized by HepSS-1 (FIG. 2). HepSS-1 recognized the N-sulfation of heparan sulfate, rather than the O-sulfation of heparan sulfate, heparin, or hyaluronic acid. Some O-SO₃- groups were spatially closely associated with NHCOCH₃ in GlcNac to form a molecular structure recognizable by HepSS-1. The results of the HepSS-1 recognition assay further showed that sHA could be recognized by HepSS-1, while both TCIs HA and R&D HA could not be recognized by HepSS-1 (referring to FIGs. 1 and 2).

### 1.3. Preparation and identification of other compounds

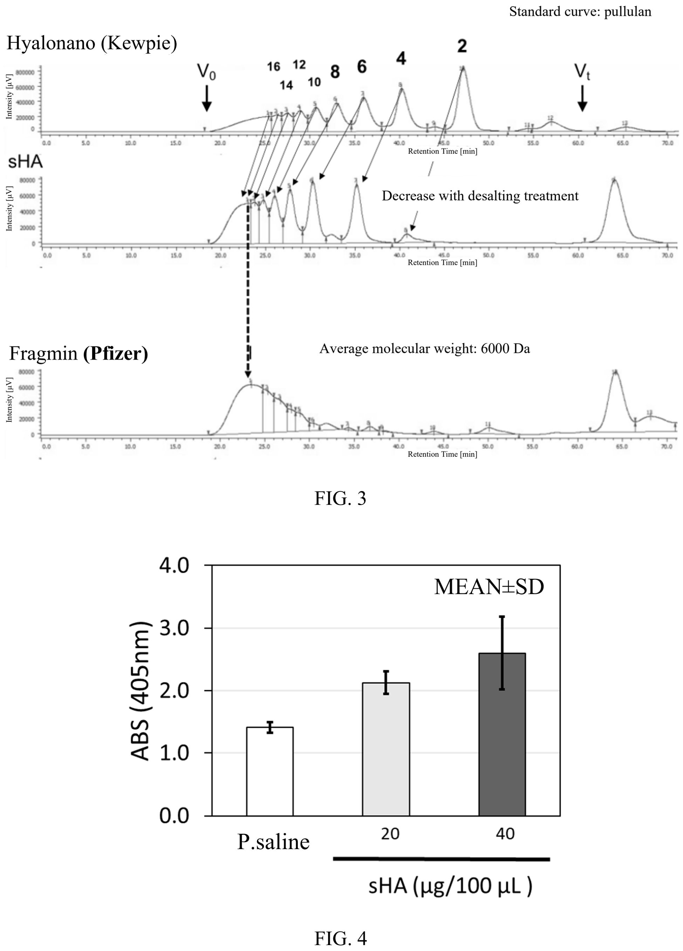

It was experimentally verified that products having similar degrees of sulfation described above (referring to FIG. 3) could also be prepared using hyaluronic acid with other molecular weights, such as hyaluronic acid with n ranging from 3-9 (8-20 mers) and a weight-average molecular weight (MW) of 2480-6590 other than n = 3 according to the process of section 1.11. Furthermore, ¹H NMR showed that when n was 3-9 (8-20 mers), the weight-average molecular weight (MW) was 2480-6590, and the degree of sulfation (i.e., the number of [SO3-] groups per disaccharide) was < 4.2 and > 3.65 (wherein the degree of sulfation of completely sulfated disaccharides at both ends was counted as 5), the compounds could all be recognized by HepSS-1.

### Example 2

### Physiological Activity of Compound of the Present Disclosure

### 2.1. Anti-SARS-CoV-2 infection assay

The binding activity of sHA of the present disclosure to the SARS-CoV-2 spike protein was assessed using an ELISA-based immunoassay. The experimental procedures are as follows:
sHA-1 (20 µg/100 µL, 20 µg/100 µL) prepared according to Example 1 was added to a 96-well plate coated with the SARS-CoV-2 spike protein;
incubation was performed with HepSS-1 (anti-N-acetyl heparan sulfate, mouse IgM), followed by addition of an anti-immunoglobulin twice;
incubation was performed with 3,3'-diaminobenzidine (DAB); and
detection was performed using an iMark plate Reader at a wavelength of 405 nm.

The experimental results (referring to FIG. 4) showed that the compound of the present disclosure could inhibit the binding of the SARS-CoV-2 spike protein to ACE2 on host cells, thereby providing anti-infective effects.

### 2.2. Effect of compound of the present disclosure on coagulation

The anticoagulant activity of the compound was assessed by measuring prothrombin time according to the reference [Sulakshan Sulakshana et al., Anesth Essays Res., 2021, 15(4): 341-347].

The experimental method and steps are as follows:
1) About 2 mL of blood was obtained from the abdominal aorta of male SD rats (8 weeks old). One sample corresponded to one rat, and n = 3.
2) Each substance was added to the blood at a concentration of 27.8 µg/mL or 278 µg/mL, and mixing was performed (generally, 27.8 µg/mL was used for transfusion).
3) To assess the anticoagulant effect of the samples, prothrombin time was measured using CoaguChek X (Sekisui Medical).

### Control group:

1) Normal saline;
2) Unfractionated heparin (UFH), sodium heparin injection 5000 units/5 mL, Mochida (average molecular weight: 15000);
3) Fragmin: low molecular weight heparin, average molecular weight of 5000, 5000 units/5 mL;
4) Arixtra: low molecular weight heparin, average molecular weight of 1728 (5 mers), 5000 units/5 mL;
5) No addition.

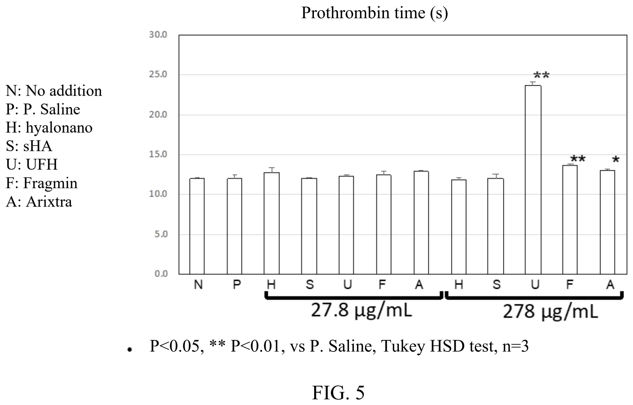

The assay results are shown in FIG. 5. At 27.8 µg/mL, none of the substances caused any change. At 278 µg/mL, the prothrombin time of the UFH, Fragmin, or Arixtra group was significantly longer than that of the normal saline group and the no-addition group. However, sHA-1 prepared according to Example 1 showed no effect. Fragmin and Arixtra are heparin drugs with low molecular weight intended to reduce anticoagulant activity. However, according to existing studies, they exhibit weak binding to the SARS-CoV-2 spike protein. sHA-1 did not exhibit anticoagulant activity even at 10 times the clinically used concentration of heparin. Therefore, compared with heparin or even low-molecular-weight heparin, the sHA of the present disclosure is less likely to cause serious adverse clinical reactions such as bleeding and thrombocytopenia.

The embodiments of the present disclosure have been described above. However, the embodiments described above are not intended to limit the present disclosure. Any modification, equivalent replacement, improvement, and the like made without departing from the spirit and principle of the present disclosure shall fall within the claimed scope of the present disclosure.

## Claims

1. A compound of formula I or a pharmaceutically acceptable salt thereof, wherein a structure of formula I is shown as follows: 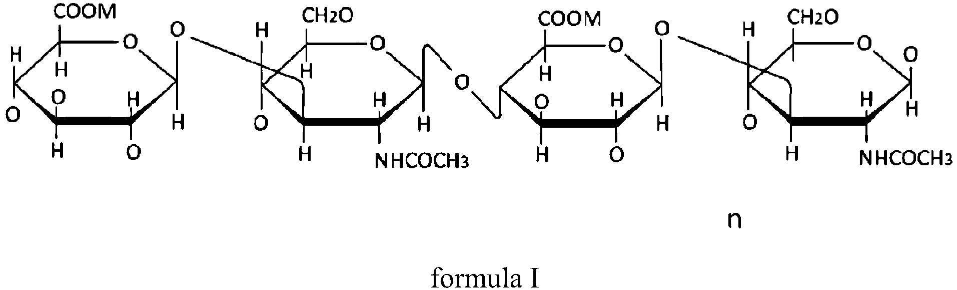
wherein in formula I, R is identical or different and is each independently selected from H and SO₃H;
M is selected from a proton and a monovalent cation;
n is selected from 3-9;
in formula I, a degree of sulfation is less than or equal to 4.2 and greater than 3.65; and the structure of formula I can be recognized by HepSS-1.

2. The compound of formula I or the pharmaceutically acceptable salt thereof according to claim 1, wherein in formula I, NHCOCH₃ is not sulfated;
preferably, n is selected from 3, 4, 5, 6, 7, 8, and 9; and
preferably, the monovalent cation is selected from Na⁺ and K⁺.

3. The compound of formula I or the pharmaceutically acceptable salt thereof according to claim 1 or 2, wherein in formula I, the degree of sulfation is less than or equal to 4.2 and greater than or equal to 3.75.

4. A preparation method for the compound of formula I or the pharmaceutically acceptable salt thereof according to any one of claims 1-3, comprising the following steps:
(1) mixing water with hyaluronic acid or a water-soluble salt thereof;
(2) adding a sulfating agent and reacting;
(3) adding ethanol, mixing, and centrifuging;
(4) removing supernatant and adding water, adding ethanol, mixing, and centrifuging;
(5) removing supernatant and allowing to stand for 4-24 hours; and
(6) adding water, filtering, and lyophilizing to obtain a product.

5. The method according to claim 4, wherein the sulfating agent is selected from pyridine coordination complexes of sulfur trioxide.

6. The method according to claim 4, wherein:
in step (1), 0.5-3 g of hyaluronic acid or a water-soluble salt thereof is mixed with each 1 mL of water.

7. The method according to any one of claims 4-6, wherein:
in step (2), the reaction is performed at a temperature of 0-20 °C, and the reaction is performed for 24-100 hours;
the sulfating agent and the hyaluronic acid or the water-soluble salt thereof are in a mass ratio of 0.5-6:100;
in step (3), an addition amount of ethanol is: 10-50 mL of ethanol per 1 g of hyaluronic acid or the water-soluble salt thereof; and
in step (4), an addition amount of water is: 0.5-2 mL of water per 1 g of hyaluronic acid or the water-soluble salt thereof.

8. A composition, comprising the compound of formula I or the pharmaceutically acceptable salt thereof according to any one of claims 1-3.

9. The composition according to claim 8, further comprising a pharmaceutically acceptable carrier.

10. Use of the compound of formula I or the pharmaceutically acceptable salt thereof according to any one of claims 1-3 or the composition according to claim 8 for the manufacturing of a medicament for treating an infection.

11. The use according to claim 10, wherein the infection is selected from an infection caused by coronavirus (COVID-19).

12. A method for treating an infection, the method comprising administering to a patient a therapeutically effective amount of the compound of formula I or the pharmaceutically acceptable salt thereof according to any one of claims 1-3 or the composition according to claim 8.
